# EUROPEAN PATENT SPECIFICATION

(11) **EP 0 432 494 B1**
(45) Date of publication and mention of the grant of the patent: **05.02.1997**
(21) Application number: 90121786.9
(22) Date of filing: 14.11.1990
(51) Int. Cl.: A01N 43/90, A61K 31/365

(54) **Pour-on formulations effective for the control of internal and external parasites of homothermic animals**
"Pour-on"-Formulierungen zur Bekämpfung von internen und externen Parasiten bei homoöthermen Tieren
Formulations "pour-on" efficaces dans la lutte contre les parasites internes et externes d'animaux homéothermes

(30) Priority: 15.12.1989 US 451472
(43) Date of publication of application: 19.06.1991
(73) Proprietor: AMERICAN CYANAMID COMPANY, Wayne, NJ 07470-8426 (US)
(72) Inventor: Wood, Irwin Boyden, Yardley, Pennsylvania 19067 (US); Quinlan, James, Trenton, New Jersey 08619 (US)
(74) Representative: Wächtershäuser, Günter, Prof. Dr.

(56) References cited:
- EP-A- 0 259 779
- EP-A- 0 311 180
- EP-A- 0 329 460
- GB-A- 2 088 212
- GB-A- 2 094 626
- S.P. PARKER: "Dictionary of Scientific and Technical Terms", 4th edition, 1989, pages 1023,1212, McGraw-Hill Book Co., New York, US
- CHEMICAL ABSTRACTS, vol. 111, no. 17, 23rd October 1989, page 270, abstract no. 148925m, Columbus, Ohio, US; & JP-A-01 025 706 (SANKYO CO., LTD) 27-01-1989

## Description

Pour-on formulations have been utilized by the animal industry as a means for administering certain anthelmintic agents and veterinary medicines to animals. In practice, the medicated formulation is applied directly to the external skin or hide of the animal. As such, some practitioners have extended the term pour-on to include formulations which may be dispersed in water and applied as aqueous dips, baths or sprays. In the present specification, however, the term "pour-on" is not intended to suggest this type of application. Rather, the formulation of this invention is a non-aqueous formulation which is applied with the assistance of a suitable device such as a measuring cup or squirt bottle or an automatic microspray device which permits directed application of a small amount of formulation onto the skin of the animal being treated.

While a number of anthelmintic agents and veterinary medicines such as tetramisole, levamisole, trichlorphon and fenthion, have been successfully prepared as pour-on compositions, all veterinary medicines do not lend themselves to such formulation. Moreover, even those medicines that have been so formulated generally have been found to be significantly less effective as pour-on formulations than they are when administered orally or parenterally.

For successful formulation as a pour-on a drug must be active when dissolved, dispersed or emulsified in a suitable solvent which is well tolerated by the animals skin. The drug must be readily adsorbable through the animals skin and the composition as a whole should disperse or spread well over the animals body and be relatively non-viscid when so spread. It must also be recognized that each drug has its own chemical and physical properties that require special consideration and limit the types of solvents, diluents, stabilizing agents and other formulatory agents that can be employed in the formulation of that particular drug or medicine.

EP-A-0 311 180 discloses compositions and methods for controlling external parasites such as ticks, flies, lice and keds. However, this reference does not disclose a formulation which may be used for controlling internal parasites by an application to the skin or hide of an animal.

GB-A-2,088,212 discloses compositions for controlling sheep ectoparasites. The problem of controlling endoparasites with a pour-on formulation is not addressed in this reference.

It is, therefore, an object of the present invention to provide a non-irritating pour-on formulation which is effective for the control of insects and internal and external parasitic infections and infestations of farm and companion animals.

It is also an object of this invention to provide an effective non-aqueous pour-on formulation that contains, as the active ingredient, an antibiotic selected from LL-F28249α, a 23-oxo or 23-imino derivative of LL-F28249α-λ, a milbemycin molecule or an avermectin molecule, which exhibits excellent penetration of the animals hide or skin, spreads well and rapidly over the animals body, is non-malodorous, non-irritating and relatively non-viscid toward dust, dirt and foreign matter encountered by the treated animals.

It is a further object of this invention to provide a method for treating, controlling, preventing or protecting quadruped farm and companion animals from infestation and infection by helminths, acarids and arthropod endo- and ectoparasitic insects by topically applying to said animals a pour-on formulation containing an anthelmintically, acaricidally or arthropod endo- or ectoparasiticidally effective amount of the antibiotic LL-F28249α or a 23-oxo or 23-imino derivative of LL-F28249α.

The present invention provides a non-aqueous pour-on composition for combating helminth, acarid and arthropod endo- and ectoparasitic insect infestations and infections of farm and companion animals characterized by an anthelmintically, acaricidally or an arthropod endo- or ectoparasitic insecticidally effective amount of a compound selected from the group consisting of LL-F28249α-λ, a 23-oxo (keto) or 23-imino derivative of LL-F28249α-λ, a milbemycin molecule and an avermectin molecule, dissolved or dispersed in a mixture comprising: (a) from 5.0% to 15% w/v of an aromatic solvent having a mixed aniline point of from 13.4°-15.4°C, a Kauri-butanol value of 92 and a specific gravity @ 15.6°/15.6°C of from 0.875 to 0.899; from about 2.0% w/v to 8.0% w/v of PPG-2 Myristyl ether propionate; from 0 w/v to about 15.0% w/v of polybutenes having a number average molecular weight of from 320 to 2300 and a Cleveland open cup flash point of from 154°C to 307°C; and quantity sufficient to 100% w/v of a pharmacologically acceptable oil,

The compounds designated LL-F28249α-λ are (collectively) isolates from the fermentation broth of the microorganism Streptomyces cyaneogriseus subspecies noncyanogenus, deposited in the NRRL under deposit accession No. 15773. The method for preparation of LL-F28249α is disclosed in United States Patent application Serial No. 732,252, filed May 10, 1985 (published as US-A-5106994 and EP-A-0170006).

The 23-oxo (keto) and 23-imino derivatives of LL-F28249α-λ compounds, useful in the pour-on formulations of this invention, are disclosed in United States Patent 4,916,154 issued April 10, 1990. Although United States Patent 4,916,154 indicates that the 23-oxo (keto) and 23-imino derivatives of LL-F28249α-λ may be administered by pouring on the skin of an animal via a solution, it only suggests that the active compound may be dissolved in dimethylsulfoxide, propylene glycol or the like or in a combination of solvents. These formulations may not be entirely satisfactory since the use of dimethylsulfoxide may cause instability of the 23-(oxo) or 23-imino derivatives of LL-F28249α-λ and/or leave a molorodous scent on the treated animal. The propylene glycol compositions may tend to be sticky and collect dust and dirt and debris from the animals' surroundings.

The pour-on veterinary formulations of this invention have the following compositions:

| | % w/v | Compound |
|---|---|---|
| (A) | 0.1 - 5.0 | active ingredient |
| | 5.0 - 15.0 | aromatic solvent (Kauri-butanol value-92) |
| | 2.0 - 8.0 | PPG-2 Myristyl Ether propionate (spreader) |
| | 0 - 15.0 | Polybutene (number average Molecular weight range from 320 to 2300) |
| | QS to 100% | Mineral or vegetable oil; |

These compositions may be prepared by dissolving, dispersing or emulsifying about 0.1% to 5.0% w/v of the active ingredient, i.e. a compound selected from the group consisting of LL-F28249α-λ, a 23-oxo or 23-imino derivative of said compound LL-F28249α-λ, a milbemycin molecule or an avermectin molecule, in a mixture consisting essentially of 5.0% to 15.0% w/v of an aromatic solvent having a Kauri-butanol value of 92, a mixed aniline point between 13.4°C and 15.4°C and a specific gravity @ 15.6°/15.6°C of 0.875-0.899; 2.0% to 8.0% w/v of PPG-2 myristyl ether propionate; 0% to 15.0% w/v of a polybutene having a number average molecular weight range of 320 to 2300 and the remainder of the mixture a pharmacologically acceptable mineral or vegetable oil.

Advantageously, the above compositions are well tolerated by the animals and non-damaging to the animals' skin, hide or hair. The formulations are non-malodorous. They spread well and rapidly over the animal's body and the active ingredient thereof is found to be readily absorbed through the hide or skin of the treated animals.

The preferred active ingredients useful in the preparation of the compositions of this invention have the following structures:
23-(O-methyloxime)-F28249α 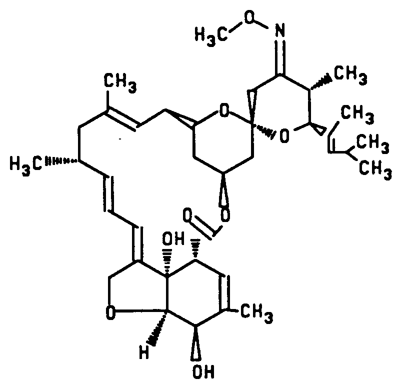 and
F28249α

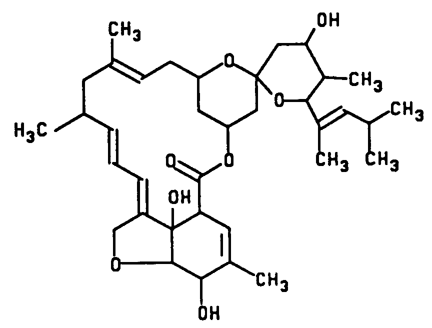

The compositions of this invention are highly effective for protecting or treating farm and companion animals, particularly quadrupeds such as cattle, sheep, horses, swine, goats, dogs, cats and the like, against infection and infestation by helminths, nematodes, acarids and arthropod endo- and ectoparasitic insects.

Helminthiases is a widespread disease found in many farm and companion animals and responsible for significant economic losses throughout the world. Among the helminths most frequently encountered are the group of worms referred to as nematodes. The nematodes are found in the intestinal tract, heart, lungs, blood vessels and other body tissues of animals and are a primary cause of anemia, weight loss and malnutrition in the infected animals. They do serious damage to the walls and tissue of the organs in which they reside and, if left untreated, may result in death to the infected animals.

The nematodes most commonly found to be the infecting agents of animals include Haemonchus and Ostertagia generally found in the stomach; Cooperia, Oesphagostonum and Nematodirus generally found in the intestinal tract and Dictyocaulus found in the lungs. Treatment of animals to prevent infestation thereof by the above nematodes or to reduce or control the proliferation of these infecting agents in animals is thus an important and desirable advantage of the present invention.

Besides controlling helminths and nematodes, the present invention also controls several arthropod endo-parasitic infestations such as cattle grub infestations.

It has been further found that acarid and arthropod ectoparasitic insect infestations may be controlled, prevented or eliminated by applying to said animals an acaricidally or ectoparasiticidally effective amount of the above-described LL-F28249 compound or derivative thereof or milbemycin or avermectin molecule. This may be achieved by applying the active amount to the skin, hide and/or hair of the animals, usually in the form of a liquid formulated composition in sufficient amount to provide the treated animal with about 0.1 mg to 5.0 mg of active compound per kg of animal body weight. In practice it has been found that generally 0.2 mg to 2.0 mg of LL-F28249α or 23-(O-methyloxime)-F28249α is sufficient to control helminths such as Ostertagia circumcincta, Haemonchus contortus and Trichostrongylus colubriformis.

It has also been found that the pour-on composition disclosed in this application which contains, as the active ingredient, 23-(O-methyloxime)F28249α provides excellent control of the Biting Louse, Damalina ovis. Moreover, it has been found that this formulation which contains 23-(O-methyloxime)F28249α is unique in its ability to provide superior control of Psoroptic mange on animals, such as cattle and sheep.

The present invention is further delineated by the examples set forth below which are provided simply by way of illustration and not intended to be limitations of the invention.

### EXAMPLE 1

### Evaluation of 13-(O-Methyloxime)-F28249α for control of Psoroptic Mange, Psoroptes ovis, on cattle

The pour-on formulation of the present invention is prepared by blending together 0.5% w/v of 23-(O-methyloxime)-F28249α; 10% w/v of an aromatic solvent having (1) a mixed aniline point of 15.4°C (test method ASTM D 611), (2) Kauri-butanol value 92, (3) specific gravity @ 15.6°/15.6°C of 0.899, (4) viscosity, CP @ 25°C 1.20 and (5) a mass composition aromatic C₉-8% w/v, C₁₀-74%, C₁₁-15% and C₁₂-1%; 5% w/v of the emollient ester PPG-2-myristyl ether propionate, (spreader) melting point -5°C; and 84.5% w/v of light mineral oil.

Calves with naturally acquired infestations of Psoroptic mange are randomly selected weighed and taged. The calves are then treated by pouring the test formulation down the midline of the back of test animals at volumes providing 10 ml of formulation per 100 kg of live animal body weight or 0.5 mg of active ingredient per kg live weight. The treated calves are placed in pens and provided feed and water ad libitum. The calves are inspected on days 7 and 14 after treatment for the presence of psoroptic mange live mites. Data obtained are reported below.

**Table I**

| Evaluation of 23-(O-methyloxime)-F28249alpha formulation for controlling Psoroptic mange on cattle | | | | | |
|---|---|---|---|---|---|
| Treatment | Initial² Infestation | Live weight (kg) | Total Dose ml | Effect of Posttreatment | |
| | | | | Day 7 | Day 14 |
| 23-(O-methyloxime) -F28249alpha pour-on formulation | +++ | 170 | 17 | mange at one site | no mites healthy skin^{b} |
| 23-(O-methyloxime) -F28249alpha pour-on formulation | ++ | 150 | 15 | two mites | no mites^{b} healthy skin |
| 23-(O-methyloxime) -F28249alpha pour-on formulation | ± | 170 | 17 | no mites | no mites^{b} healthy skin |
| untreated control | *** | 150 | 0 | no change^{c} | -- |
| untreated control | ± | 160 | 0 | no change | no change |

| | | | | | |
|---|---|---|---|---|---|
| ^{a}Assessment of aternal infestation +++ = high ++ = moderate ± = very light | | | | | |
| ^{b} Cured | | | | | |
| ^{c} treated with commercial product to cure mange to save animal. | | | | | |

### EXAMPLE 2

### Preparation of pour-on Formulations containing 23-(O-Methyloxime)-F28249α for controlling Gastrointestinal Nematodes and the Biting Louse of Sheep

For the evaluations reported in Example 3, the following formulations are prepared:
1)
   (a) 0.5% w/v; 23-(O-methyloxime)-F28249α,
   (b) 10.0% w/v aromatic solvent sp. gravity @ 15.6°/15.6°C=0.899, mixed aniline point 15.4°C, Kauri-butanol valve 92;
   (c) 5.0% w/v PPG-2-myristyl ether propionate (spreader),
   (d) 84.5% light mineral oil;
2)
   (a) 0.2% w/v 23-(O-methyloxime)-F28249α,
   (b) 10.0% w/v aromatic solvent sp. gravity @ 15.6°/15.6°C=0.899, mixed aniline point 15.4°C and Kauri-butanol value 92,
   (c) 5.0% w/v PPG-2-myristyl ether propionate,
   (d) 84.8% light mineral oil;
3)
   (a) 0.5% w/v 23-(O-methyloxime)-F28249α,
   (b) 10.0% w/v aromatic solvent sp. gravity @ 15.6°/15.6°C=0.899, mixed aniline point 15.4°C and Kauri-butanol value 92,
   (c) 5.0% w/v PPG-2-myristyl ether propionate,
   (d) 74.5% light mineral oil;
   (e) 10.0% w/v polybutene H-1900 kinematic viscosity at 99°C=4069-4382 Flash point open cup = 243°C Specific gravity at 15.5°C = .898-.916 Molecular weight number average = 2300; (agent promoting adhesion and water repellency)
4)
   (a) 0.2% w/v 23-(O-methyloxime)-F28249α,
   (b) 10.0% w/v aromatic solvent sp. gravity @ 15.6°/15.6°C=0.899, mixed aniline point 15.4°C and Kauri-butanol value 92,
   (c) 5.0% w/v PPG-2-myristyl ether propionate,
   (d) 74.8% light mineral oil;
   (e) 10.0% w/v polybutene H 1900 kinematic viscosity at 99°C = 4069-4382 Flash point open cup = 243°C Specific gravity at 15.5°C = .898-.916 Molecular weight member average = 2300;

### EXAMPLE 3

### Evaluation of pour-on formulations containing 23-(O-methyloxime)-F28249α for controlling gastrointestinal nematodes in sheep

Merino sheep are given artificially-induced infestations of Damalina ovis the biting louse and artificially-induced infections of the gastrointestinal nematodes Ostertagia circumcincta, Haemonchus contortus and Trichostronglyus colubriformis. The sheep are then sheared, weighed and placed in individual pens. The infected sheep are then treated with the selected formulation by pouring the formulation down the midline of the sheeps' back. The formulations are then applied to the weighed sheep in sufficient amount to provide from 0.2 to 1.0 mg/kg of live animal body weight. Fourteen days after treatment, three sheep from each treatment and the control group are necropsied and the nematodes recovered and counted. Data obtained are reported in Table 2 below where it can be seen that all formulations are highly effective against the abomasol nematodes Ostertagio circumcincta and Haemonchus contortus at doses of 0.2 mg/kg and 0.5 mg/kg or 1 mg/kg when poured along the midline on the backs of off-sheared sheep. It can also be seen that doses of from 0.5 mg/kg to 1 mg/kg of the 23-(O-methyloxime)-F28249α provided in the above-said formulations are effective for controlling the intestinal bankrupt worm, Trichostrongylus colubriformis. In these tests nematode egg counts dropped to nearly zero after treatment and remained that way to the end of the trial.

From Table 3 below, it can also be seen that the pour-on formulations are highly effective against the biting louse, Damalina ovis, throughout the six week trial period.

**Table 2**

| Efficacy of 23-(O-methyloxime)-F28249α Pour-on Against Nematodes in Sheep | | | | | |
|---|---|---|---|---|---|
| Formulation | Dose mg/kg | Dosage ml/10 kg | Ostertagia circumcincta | Haemonchus contortus | Trichostrongylus colubriformis |
| Control | 0 | 0 | 2025* | 428* | 1375* |
| | | | % Efficacy | | |
| 1 | 1.0 | 2 | 100 | 100 | 99.6 |
| 1 | 0.5 | 1 | 100 | 100 | 72 |
| 2 | 0.2 | 1 | 96 | 89 | 24 |
| 3 | 1.0 | 2 | 100 | 100 | 89 |
| 3 | 0.5 | 1 | 100 | 100 | 76 |
| 4 | 0.2 | 1 | 100 | 100 | 17 |

| | | | | | |
|---|---|---|---|---|---|
| *Arithmetic Mean Adult Worm Count, 14 days posttreatment The worm egg counts of the remaining sheep were markedly reduced from the initial levels at the time of treatment at the end of the six-week trial period. | | | | | |

**Table 3**

| Efficacy of 23-(O-methyloxime)-F28249alpha Pour-On Against the Biting Louse, Damalina ovis, on Sheep | | | | | | |
|---|---|---|---|---|---|---|
| Formulation | Dose mg/kg | Dosage ml/kg | Average No. of Lice | | | |
| | | | 0 | 2 Weeks | 4 Weeks | 6 Weeks |
| Control | | | 16.3 | 10.3 | 8.7 | 8 |
| 1 | 1.0 | 2 | 46.3 | 0.67 | 0 | 0 |
| 1 | 0.5 | 1 | 30 | 1.3 | 0 | 0 |
| 2 | 0.2 | 1 | 35 | 3.3 | 1 | 1 |
| 3 | 1.0 | 2 | 27 | 0.33 | 0 | 0 |
| 3 | 0.5 | 1 | 21 | 0.67 | 0 | 0 |
| 4 | 0.2 | 1 | 18.7 | 10.3 | 4 | 1 |

## Claims (Claims for the following Contracting State(s): AT, BE, CH, LI, DE, DK, FR, GB, IT, NL, SE)

1. A non-aqueous pour-on composition for combating helminth, acarid and arthropod endo- and ectoparasitic insect infestations and infections of farm and companion animals characterized by an anthelmintically, acaricidally or an arthropod endo- or ectoparasitic insecticidally effective amount of a compound selected from the group consisting of LL-F28249α-λ, a 23-oxo (keto) or 23-imino derivative of LL-F28249α-λ, a milbemycin molecule and an avermectin molecule, dissolved or dispersed in a mixture comprising: (a) from 5.0% to 15% w/v of an aromatic solvent having a mixed aniline point of from 13.4°-15.4°C, a Kauri-butanol value of 92 and a specific gravity @ 15.6°/15.6°C of from 0.875 to 0.899; from about 2.0% w/v to 8.0% w/v of PPG-2 Myristyl ether propionate; from 0 w/v to about 15.0% w/v of polybutenes having a number average molecular weight of from 320 to 2300 and a Cleveland open cup flash point of from 154°C to 307°C; and quantity sufficient to 100% w/v of a pharmacologically acceptable oil,

2. The composition according to Claim 1, wherein the compound is 23-(O-methyloxime)-F28249α.

3. The composition according to claim 1, wherein the compound is LL-F28249α.

4. The composition according to Claim 1 characterized by 0.1% to 5.0% w/v of 23-(O-methyloxime)-F28249α; 5.0% to 15% w/v aromatic solvent; about 2.0% to 8.0% w/v PPG-2 Myristyl ether propionate; up to 15.0% w/v polybutenes and quantity sufficient to 100% w/v of mineral or vegetable oil.

## Claims (Claims for the following Contracting State(s): ES, GR)

1. A process for producing a non-aqueous pour on composition for combating helminth, acarid and arthropod endo-and ectoparasitic insect infestations and infections of farm and companion animals characterized by dissolving or dispersing an anthelmintically, acaricidally or an arthropod endo- or ectoparasitic insecticidally effective amount of a compound selected from the group consisting of LL-F28249α-λ, a 23-oxo (keto) or 23-imino derivative of LL-F28249α-λ, a milbemycin molecule and an avermectin molecule, in a mixture comprising: (a) from 5.0% to 15% w/v of an aromatic solvent having a mixed aniline point of from 13.4° - 15.4°C, a Kauri-butanol value of 92 and a specific gravity at 15.6°/15.6°C of from 0.875 to 0.899; from about 2.0% w/v to 8.0% w/v of PPG-2 Myristyl ether propionate; from 0 w/v to about 15.0% w/v of polybutenes having a number average molecular weight of from 320 to 2300 and a Cleveland open cup flash point of from 154°C to 307°C; and quantity sufficient to 100% w/v of a pharmacologically acceptable oil.

2. The process according to Claim 1, wherein the compound is 23-(O-methyloxime)-F28249α.

3. The process according to claim 1, wherein the compound is LL-F28249α.

4. The process according to Claim 1 characterized by 0.1% to 5.0% w/v of 23-(O-methyloxime)-F28249α; 5.0% to 15% w/v aromatic solvent; about 2.0% to 8.0% w/v PPG-2 Myristyl ether propionate; up to 15.0% w/v polybutenes and quantity sufficient to 100% w/v of mineral or vegetable oil.

## Patentansprüche (Patentansprüche für folgende(n) Vertragsstaat(en): AT, BE, CH, LI, DE, DK, FR, GB, IT, NL, SE)

1. Eine nichtwäßrige "Pour-on"-Zusammensetzung zur Bekämpfung von Befall und Infektionen mit internen und externen Wurm-, Milben- und Gliederfüßler-Parasiten bei landwirtschaftlichen und begleitenden Tieren, gekennzeichnet durch eine gegen internen und externen Wurm-, Milben- und Gliederfüßler-Parasiten-Befall insektizid wirksame Menge einer aus der aus LL-F28249α-λ, einem 23-Oxo-(Keto-) oder 23-Imino-Derivat von LL-F28249α-λ, einem Milbemycin-Molekül und einem Avermectin-Molekül bestehenden Gruppe ausgewählten Verbindung, die gelöst oder dispergiert ist in einer Mischung, die (a) von 5.0 % bis 15 % Gew./Vol. eines aromatischen Lösungsmittels mit einem gemischten Anilin-Punkt von 13.4°-15.4°C, einem Kauri-Butanol-Wert von 92 und einem spezifischen Gewicht bei 15.6°/15.6°C von 0.875 bis 0.899; von ungefähr 2.0 % Gew./Vol. bis 8.0 % Gew./Vol. an PPG-2-Myristyletherpropionat; von 0 % Gew./Vol. bis ungefähr 15.0 % Gew./Vol. an Polybutenen mit einem zahlengemittelten Molekulargewicht von 320 bis 2300 und einem Cleveland-Becher-Flammpunkt von 154°C bis 307°C sowie die zu 100 % Gew./Vol. ergänzende Menge eines pharmakologisch annehmbaren Öls enthält.

2. Die Zusammensetzung gemäß Anspruch 1, worin die Verbindung 23-(O-Methyloxim)-F28249α ist.

3. Die Zusammensetzung gemäß Anspruch 1, worin die Verbindung LL-F28249α ist.

4. Die Zusammensetzung gemäß Anspruch 1, gekennzeichnet durch 0.1 % bis 5.0 % Gew./Vol. an 23-(O-Methyloxim)-F28249α; 5.0 % bis 15 % Gew./Vol. aromatisches Lösungsmittel; ungefähr 2.0 % bis 8.0 % Gew./Vol. PPG-2-Myristyletherpropionat; bis zu 15 % Gew./Vol. Polybutene und die zu 100 % Gew./Vol. ergänzende Menge Mineral- oder Pflanzenöl.

## Patentansprüche (Patentansprüche für folgende(n) Vertragsstaat(en): ES, GR)

1. Ein Verfahren zur Herstellung einer nichtwäßrigen "Pour-On"-Zusammensetzung zur Bekämpfung von Befall und Infektionen mit internen und externen Wurm-, Milben- und Gliederfüßler-Parasiten bei landwirtschaftlichen und begleitenden Tieren, gekennzeichnet durch das Auflösen oder Dispergieren einer gegen internen und externen Wurm-, Milben- und Gliederfüßler-Parasiten-Befall insektizid wirksamen Menge einer aus der aus LL-F28249α-λ, einem 23-Oxo-(Keto-) oder 23-Imino-Derivat von LL-F28249α-λ, einem Milbemycin-Molekül und einem Avermectin-Molekül bestehenden Gruppe ausgewählten Verbindung in einer Mischung, die (a) von 5.0 % bis 15 % Gew./Vol. eines aromatischen Lösungsmittels mit einem gemischten Anilin-Punkt von 13.4°-15.4°C, einem Kauri-Butanol-Wert von 92 und einem spezifischen Gewicht bei 15.6°/15.6°C von 0.875 bis 0.899; von ungefähr 2.0 % Gew./Vol. bis 8.0 % Gew./Vol. an PPG-2-Myristyletherpropionat; von 0 % Gew./Vol. bis ungefähr 15.0 % Gew./Vol. an Polybutenen mit einem zahlengemittelten Molekulargewicht von 320 bis 2300 und einem Cleveland-Becher-Flammpunkt von 154°C bis 307°C sowie die zu 100 % Gew./Vol. ergänzende Menge eines pharmakologisch annehmbaren Öls enthält.

2. Das Verfahren gemäß Anspruch 1, worin die Verbindung 23-(O-Methyloxim)-F28249α ist.

3. Das Verfahren gemäß Anspruch 1, worin die Verbindung LL-F28249α ist.

4. Das Verfahren gemäß Anspruch 1, gekennzeichnet durch 0.1 % bis 5.0 % Gew./Vol. an 23-(O-Methyloxim)-F28249α; 5.0 % bis 15 % Gew./Vol. aromatisches Lösungsmittel; ungefähr 2.0 % bis 8.0 % Gew./Vol. PPG-2-Myristyletherpropionat; bis zu 15 % Gew./Vol. Polybutene und die zu 100 % Gew./Vol. ergänzende Menge Mineral- oder Pflanzenöl.

## Revendications (Revendications pour l'(les) Etat(s) contractant(s) suivant(s): AT, BE, CH, LI, DE, DK, FR, GB, IT, NL, SE)

1. Composition "pour-on" non aqueuse pour lutter contre les infestations et les infections par les helminthes, les acariens et les arthropodes constitués des insectes, endo- et ectoparasites des animaux d'élevage et des animaux de compagnie, caractérisée en ce qu'une quantité efficace contre les helminthes, les acariens ou les arthropodes constitués des insectes, endo- ou ectoparasites, d'un composé, choisi dans le groupe constitué par le LL-F28249α-λ, un dérivé 23-oxo (céto) ou 23-imino de LL-F28249α-λ, une molécule de milbémycine et une molécule d'avermectine, dissoute ou dispersée dans un mélange comprenant : (a) 5,0 à 15 % p/v d'un solvant aromatique ayant un point d'aniline composé de 13,4° à 15,4°C, un indice kauri-butanol de 92 et une densité à 15,6°/15,6°C de 0,875 à 0,899 ; environ 2,0 % p/v à 8,0 % p/v de PPG-2 Myristyl éther propionate ; 0 % p/v à environ 15,0 % p/v de polybutènes ayant une moyenne en nombre du poids moléculaire de 320 à 2 300 et un point d'éclair Cleveland en vase ouvert de 154°C à 307°C ; et le complément à 100 % p/v d'une huile pharmacologiquement acceptable.

2. Composition selon la revendication 1, dans laquelle le composé est le 23-(O-méthyloxime)-F28249α.

3. Composition selon la revendication 1, dans laquelle le composé est le LL-F28249α.

4. Composition selon la revendication 1, caractérisée en ce qu'elle contient 0,1 % à 5,0 % p/v de 23-(O-méthyloxime)-F28249α ; 5,0 % à 15 % p/v de solvant aromatique ; environ 2,0 % à 8,0 % p/v de PPG-2 Myristyl éther propionate ; jusqu'à 15,0 % p/v de polybutènes ; et le complément à 100 % p/v d'une huile minérale ou végétale.

## Revendications (Revendications pour l'(les) Etat(s) contractant(s) suivant(s): ES, GR)

1. Procédé pour la préparation d'une composition "pour-on" non aqueuse pour lutter contre les infestations et les infections par les helminthes, les acariens et les arthropodes constitués des insectes, endo- et ectoparasites des animaux d'élevage et des animaux de compagnie, caractérisé par la dissolution ou la dispersion d'une quantité efficace contre les helminthes, les acariens ou les arthropodes constitués des insectes, endo- ou ectoparasites, d'un composé, choisi dans le groupe constitué par le LL-F28249α-λ, un dérivé 23-oxo (céto) ou 23-imino de LL-F28249α-λ, une molécule de milbémycine et une molécule d'avermectine, dans un mélange comprenant : (a) 5,0 à 15 % p/v d'un solvant aromatique ayant un point d'aniline composé de 13,4° à 15,4°C, un indice kauri-butanol de 92 et une densité à 15,6°/15,6°C de 0,875 à 0,899 ; environ 2,0 % p/v à 8,0 % p/v de PPG-2 Myristyl éther propionate ; 0 % p/v à environ 15,0 % p/v de polybutènes ayant une moyenne en nombre du poids moléculaire de 320 à 2 300 et un point d'éclair Cleveland en vase ouvert de 154°C à 307°C ; et le complément à 100 % p/v d'une huile pharmacologiquement acceptable.

2. Procédé selon la revendication 1, dans lequel le composé est le 23-(O-méthyloxime)-F28249α.

3. Procédé selon la revendication 1, dans lequel le composé est le LL-F28249α.

4. Procédé selon la revendication 1, caractérisé en ce que la composition contient 0,1 % à 5,0 % p/v de 23-(O-méthyloxime)-F28249α ; 5,0 % à 15 % p/v de solvant aromatique ; environ 2,0 % à 8,0 % p/v de PPG-2 Myristyl éther propionate ; jusqu'à 15,0 % p/v de polybutènes ; et le complément à 100 % p/v d'une huile minérale ou végétale.
